(19) Europäisches Patentamt
European Patent Office
Office européen des brevets

(11) **EP 3 203 208 B1**

(12) **EUROPÄISCHE PATENTSCHRIFT**

(45) Veröffentlichungstag und Bekanntmachung des Hinweises auf die Patenterteilung:
**16.08.2023 Patentblatt 2023/33**

(21) Anmeldenummer: **17151709.7**

(22) Anmeldetag: **17.01.2017**

(51) Internationale Patentklassifikation (IPC):
**G01N 3/60** (2006.01)        **G01N 33/38** (2006.01)

(52) Gemeinsame Patentklassifikation (CPC):
**G01N 25/72; G01N 3/60; G01N 33/388;**
G01N 2203/0057; G01N 2203/0062

(54) **VERFAHREN ZUR PRUEFUNG EINER KERAMISCHEN KOMPONENTE**

METHOD FOR INSPECTING A CERAMIC COMPONENT

PROCÉDÉ DE CONTRÔLE D'UN COMPOSANT EN CÉRAMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.02.2016 DE 102016201647**

(43) Veröffentlichungstag der Anmeldung:
**09.08.2017 Patentblatt 2017/32**

(73) Patentinhaber: **Aktiebolaget SKF**
**415 50 Göteborg (SE)**

(72) Erfinder: **SCHOEPPL, Oskar**
**4020 Linz (AT)**

(74) Vertreter: **Kuhstrebe, Jochen et al**
**SKF GmbH**
**Gunnar-Wester-Straße 12**
**97421 Schweinfurt (DE)**

(56) Entgegenhaltungen:
**WO-A1-2016/023055     JP-A- H0 886 731**

• **Anonymous: "Bruchzähigkeit - Wikipedia", , 31 January 2020 (2020-01-31), XP055664006, Retrieved from the Internet: URL:https://de.wikipedia.org/wiki/Bruchzäh igkeit [retrieved on 2020-01-31]**

**Beschreibung**

Die Erfindung bezieht sich auf ein Verfahren zur Prüfung einer keramischen Komponente auf eine Einsatztauglichkeit.

Keramische Komponenten werden in einer Vielzahl von Anwendungen eingesetzt, beispielsweise in Wälzlagern, Gleitlagern, Walzen, als Schneidelement, als Implantat oder dergleichen. Oft wirken auf die Komponenten hohe mechanische Belastungen oder Temperaturen, beispielsweise an der Oberfläche.

Unter Umständen kann die Komponente an der Oberfläche, aber auch in anderen Bereichen, Risse oder Fehler aufweisen. Diese Defekte können aus unterschiedlichen Gründen vorhanden sein, beispielsweise bei einer Herstellung, einer Nachbearbeitung, wie Schleifen, Honen oder dergleichen entstanden sein. Durch Spannungen können die Fehlstellen dazu führen, dass die keramische Komponente versagt. Der Riss kann unter einer Belastung wachsen und daher zu einem unerwünschten Materialversagen führen.

Um dies zu verhindern gibt es konventionelle Prüfverfahren, mit denen fehlerhafte Bauteile aussortiert werden sollen. Beispielsweise werden die Bauteile dazu, wie in der DE 10 2010 017 351 A1 vorgeschlagen, einer Thermoschockbehandlung unterzogen. Dazu werden die keramischen Bauteile innerhalb einer kurzen Zeit einer schnellen Erhitzung ausgesetzt, um fehlerhafte Bauteile gezielt zum Ausfall zu bringen. Eine Detektion von auftretenden Rissen erfolgt während eines Abkühlens akustisch. Wird ein Wachsen eines Risses detektiert, wird das Bauteil ausgeschieden. Allerdings kann der Prüfversuch unter ungünstigen Umständen relativ unspezifisch sein. Es kann eventuell vorkommen, dass auch Bauteile ausgeschieden werden, die brauchbar sind, weil zwischen kritischen und nicht kritischen Rissen nicht unterschieden wird.

Ferner wird in dem Dokument JP H08 86731 A ein Verfahren zum Einstellen einer geeigneten Temperaturdifferenz bei einem Thermoschocktest während des Testens von Formteilen beschrieben. Dabei wird ein Vorriss in der Oberfläche einer Probe unter Verwendung einer Vickers-Vertiefung gebildet, und das Fortschreiten des Vorrisses während des Aufbringens von Wärmeschocks wird wiederholt mit einer allmählich ansteigenden Temperaturdifferenz beobachtet.

Es besteht daher ein Bedarf daran, ein Konzept für ein Prüfverfahren für eine keramische Komponente zu verbessern. Diesem Bedarf tragen das Verfahren und die Komponente nach den unabhängigen Ansprüchen Rechnung.

Ausführungsbeispiele betreffen ein Verfahren zur Prüfung einer keramischen Komponente auf eine Einsatztauglichkeit. Dazu wird die Komponente auf eine erste Temperatur temperiert. Anschließend wird die Komponente auf eine zweite Temperatur temperiert. Eine Temperaturdifferenz zwischen der ersten Temperatur und der zweiten Temperatur wird auf Basis einer minimalen Risszähigkeit ermittelt. Anschließend wird die Komponente auf Risse überprüft.

Bei der minimalen Risszähigkeit handelt es sich um einen von einem Benutzer oder Anwender vorgegebenen Wert, den die Komponente aufweisen soll, um den Einsatzzweck zu erfüllen.

Bei dem Temperieren kann es sich beispielsweise um ein Erwärmen oder ein Abkühlen der Komponente handeln. Unter Umständen kann die zweite Temperatur kleiner sein als die erste Temperatur. Das Temperieren kann beispielsweise in einem Gas zum Beispiel in Luft, beispielsweise in einem Ofen und/oder in einer Flüssigkeit, beispielsweise in einem Bad, aber auch mit Strahlung, beispielsweise mit einem Laser erfolgen. Eventuell kann es sich bei dem ersten Temperieren um ein Erwärmen handeln, das in einem Ofen ausgeführt wird und bei dem zweiten Temperieren um ein Abkühlen, das in einem Flüssigkeitsbad, beispielsweise in Wasser oder in Öl ausgeführt wird. Ein Temperieren, beispielsweise ein Erwärmen, aber auch ein Abkühlen kann dabei unter beliebig einstellbarer Atmosphäre, aber auch unter Vakuum erfolgen.

Ein Überprüfen der Komponente auf Risse kann auf jedwede Art und Weise erfolgen, die geeignet ist, um überhaupt Risse oder kritische Risse zu entdecken, beispielsweise optischem, akustische und/oder haptisch. Optisch kann zum Beispiel bedeuten, dass der Riss mit freiem Auge erkennbar oder mit einer Kamera detektierbar ist. Erfindungsgemäß wird ein Grenzwert für eine Größe festgelegt, ab der ein Riss als kritisch betrachtet wird. Bei dem Grenzwert handelt es sich um eine Länge, eine Breite und/oder eine Tiefe des Risses. Ein Grenzwert für die Größe kann beispielsweise wenigstens 0,01 mm, ein Grenzwert für die Tiefe beispielsweise wenigstens 0,01 mm und/oder ein Grenzwert für die Breite beispielsweise wenigstens 0,01 mm betragen. Ergänzend oder alternativ können auch Detektionsverfahren eingesetzt werden, mit denen nur Risse detektiert werden können, die den entsprechenden Grenzwert überschreiten. Bei einem Riss kann es sich beispielsweise um eine Fehlstelle handeln, die von einer gewünschten Beschaffenheit der Komponente abweicht. Diese Fehlstelle kann bei der Thermoschockbehandlung entstanden oder aber auch schon vor der Thermoschockbehandlung vorhanden gewesen sein. Eventuell kann die Fehlstelle oder der Riss erst durch die Thermoschockbehandlung eine kritische Größe überschritten haben. Unter einer Thermoschockbehandlung wird zum Beispiel eine spontane, also in kurzer Zeit erfolgende Temperaturerhöhung oder Abkühlung eines keramischen Bauteils verstanden. Eine kurze Zeitdauer kann beispielsweise kürzer sein als 1 Stunde, 50 min oder 30 min und/oder wenigstens 10 sec, 20 sec oder 30 sec dauern.

Bei der keramischen Komponente kann es sich um alle möglichen Bauteile handeln, die zumindest teilweise einen keramischen Werkstoff umfassen oder aus diesem hergestellt sind, beispielsweise ein Implantat für einen menschlichen oder tierischen Körper, zum Beispiel ein Gelenk, eine Knochenschraube, eine Zahnprothesen, eine Zahnbrücke oder dergleichen. Des Weiteren kann es sich bei der Komponente um eine Lagerkomponente, für ein Wälz- oder ein Gleitlager handeln, beispielsweise einen Lagerring oder einen Wälzkörper bzw. im Allgemeinen für Wälz-, Roll- und Gleitanwendungen geeignet sein. Darüber hinaus kann auch die Komponenten zum Beispiel ein Ventil, ein Düsenkörper, ein Werkzeugteil, ein Schneidelement, eine keramische Leiterplatte, eine funktionelle Komponente oder dergleichen sein. Manche der Komponenten können dabei auch metallische oder organische Materialien enthalten. Bei allen diesen Bauteilen kann ein Versagen unerwünscht sein und zu erheblichen Nacharbeiten und gegebenenfalls Operationen führen. Bei manchen Ausführungsbeispielen kann durch das Verfahren oder den Einsatz von Komponenten, die mit dem Verfahren geprüft wurden, zumindest die Gefahr, dass ein Versagen eintritt, reduziert werden.

Eine minimale Risszähigkeit kann dabei beispielsweise basierend auf Erfahrungswerten, Versuchen oder dergleichen bestimmt werden. Eine minimale geforderte Risszähigkeit kann beispielsweise wenigstens 4.0 MPa m$^{1/2}$ betragen.

Die Temperaturdifferenz kann beispielsweise ausgehend von dem Griffith/ Irwin-Kriterium bestimmt werden:

$$K \geq K_{Ic} \text{ mit } K = \sigma_{Ref} \, Y \, \sqrt{a\pi} \qquad (1)$$

K steht dabei für den Spannungsintensitätsfaktor. $K_{Ic}$ stellt den kritischen Spannungsintensitätsfaktor dar. $\sigma_{Ref}$ steht für eine Referenzspannung in einer Probe ohne Riss. a steht für die Größe des Risses und Y stellt einen geometrischen Faktor dar, mit welchem die Geometrie des Risses, des Spannungsfeldes und des Probenkörpers berücksichtigt wird.

Die thermisch induzierte Spannung $\sigma_{th}$ bzw. deren Maximalwert $\sigma_{th,max}$ an einer unter Zugspannung stehenden Oberfläche kann allgemein mit der folgenden Gleichung dargestellt werden:

$$\sigma_{th,max} = \hat{\sigma}^{*}_{th} \frac{\alpha E}{1-\nu}\Delta T \qquad (2)$$

$\alpha$ ist der lineare thermische Ausdehnungskoeffizient, E steht für den E-Modul, v für das Poisson-Verhältnis und $\Delta T$ für die Temperaturdifferenz bei der Thermoschockbehandlung. Der dimensionslose Faktor $\hat{\sigma}^{*}_{th}$ bezieht sich auf die Bauteilgeometrie und einen Quenchparameter. Dieser Faktor liegt zwischen dem Wert 0 bei sehr sanftem Abkühlen und dem Wert 1 für einen sehr schnellen Abschreckprozess.

Der Spannungsintensitätsfaktor K lässt sich für den konkreten Versuch bei Vorliegen eines Risses mit der Länge a wie folgt darstellen:

$$K = \Delta T_c \frac{\alpha \text{eff } E}{1-\nu} \sigma^{*}_{th,max} Y_{max}\sqrt{a\pi} \qquad (3)$$

$Y_{max}$ ist dabei das Maximum des Geometriefaktors Y in einem bestimmten Punkt des Risses. Dieser ist von der Rissform und Position abhängig und kann durch eine Parameterstudie bestimmt werden. Zur Verlängerung eines Risses bzw. eines Defektes kommt es dann, wenn am Ort des Risses bzw. Defektes entsprechend Gleichung (3) die Risszähigkeit K des Materials erreicht wird. Durch Umformulierung der vorstehenden Gleichung (3) ergibt sich für idealisierte Bedingungen wie temperaturunabhängige Werkstoffparameter und geringe Spannungsänderungen über den Rissverlauf, dass eine bestimmte kritische Rissgröße bei vorgegebener Probengeometrie und geforderter Risszähigkeit einer bestimmten kritischen Temperaturdifferenz $\Delta T_c$ bei der Thermoschockbehandlung entspricht. Unter nicht idealisierten Bedingungen lässt sich diese Temperaturdifferenz numerisch berechnen. Wird die Temperaturdifferenz entsprechend eingestellt, kann bei manchen Ausführungsbeispielen sichergestellt werden, dass alle Komponenten, die der Thermoschockbehandlung mit der gegebenen Temperaturdifferenz standhalten, für den Einsatz im Betrieb geeignet sind bzw. die minimale geforderte Risszähigkeit aufweisen. Komponenten, bei denen bei der Prüfung Risse auftreten, die einen Grenzwert überschreiten, können aussortiert werden.

Ergänzend oder alternativ kann unter Berücksichtigung der bauteilspezifischen und systematischen Fehler für die Bestimmung der Risszähigkeit an Wälzlagerkugeln aus Siliciumnitrid der abgeschätzte Messfehler für die ermittelte Risszähigkeit maximal im Bereich von +-10 % liegen. Bei manchen Ausführungsbeispielen können die mit dem Verfahren geprüften Komponenten eine ausreichende Sicherheit gegen Versagen aufweisen.

Ergänzend oder alternativ kann ein Wert für die Risszähigkeit aus einer Tabelle entnommen und/oder experimentell ermittelt werden.

Um eine Risszähigkeit experimentell zu ermitteln, kann beispielsweise eine Risszähigkeit an einer sogenannten Probenkomponente mit dem folgenden Verfahren ermittelt werden.

Beispielsweise kann die Probenkomponente dieselben Form, Größen und Materialeigenschaften wie die keramische Komponente aufweisen.

Hierfür wird ein semi-elliptischer Oberflächenriss mit beispielsweise einem Knoop Indenter zwecks guter Reproduzierbarkeit der Rissgeometrie eingebracht. In untenstehender Tabelle wurde an Siliciumnitrid Wälzlagerkugeln mit verschiedenem Durchmesser D ein Knoop Indent mit der Last 10 und 7 kg eingebracht (HK10, HK7). Der an der Bauteiloberfläche plastisch verformte Werkstoff wird vorzugsweise durch Abtragen einer dünnen Oberflächenschicht entfernt um den Einfluss von eingebrachten Eigenspannungen auf das Messergebnis gering zu halten. Die abgetragene Schicht sollte zumindest 1/6 der längeren Diagonale des Knoop Indents betragen. Durch Einbringen einer spezifischen Temperaturdifferenz $\Delta T$ im Thermoschockexperiment erhält man eine Risszähigkeit Ksurv, wo gerade noch kein Risswachstum erkennbar ist. Bei weiterer Erhöhung des $\Delta T$ kommt es zu Risswachstum bei der Risszähigkeit Kfrac. Hier wurde die kritische Risszähigkeit Kc bereits überschritten, die mit $\Delta Tc$ entsprechend Gleichung (3) korreliert. Der Wärmeübergangskoeffizient hf beim Abschrecken von Keramiken in Wasser liegt erfahrungsgemäß im Bereich von 75000 bis 100000 $Wm^{-2}K^{-1}$. Bi ist eine dimensionslose Biotzahl aus der Modellierung des Temperaturfelds der Kugel. Die auf diese Art und Weise ermittelte Risszähigkeit kann beispielsweis auch als gemessen Risszähigkeit bezeichnet werden.

| | $h_f = 75000\ Wm^{-2}K^{-1}$ | | | $h_f = 100000\ Wm^{-2}K^{-1}$ | | |
|---|---|---|---|---|---|---|
| | Bi | Ksurv | Kfrac | Bi | Ksurv | Kfrac |
| | - | $MPa\ m^{1/2}$ | $MPa\ m^{1/2}$ | - | $MPa\ m^{1/2}$ | $MPa\ m^{1/2}$ |
| Set 1, D=12.7 mm, HK10 | 22.0 | 5.3 ± 0.3 | 5.5 ± 0.3 | 29.3 | 5.7 ± 0.3 | 5.9 ± 0.3 |
| Set 2, D=5.55 mm, HK10 | 10.2 | 5.5 ± 0.3 | 5.9 ± 0.3 | 13.7 | 6.0 ± 0.3 | 6.4 ± 0.3 |
| Set 3, D=5.55 mm, HK7 | 10.6 | 5.7 ± 0.1 | 6.0 ± 0.2 | 14.2 | 6.2 ± 0.1 | 6.5 ± 0.2 |

Bei manchen Ausführungsbeispielen ist die Ermittlung der Risszähigkeit in das Verfahren zur Prüfung integriert. Eventuell können dadurch bei manchen Ausführungsbeispielen genauere Ergebnisse ermittelt werden. Beispielsweise kann das Ermitteln der Risszähigkeit in derselben Vorrichtung zum Temperieren, an demselben Ort und/oder in einem zeitlichen Abstand, der maximal 1/10 einer Gesamtlänge des Verfahrens zu Prüfung aufweist, erfolgen. Beispielsweise kann ein Anteil einer Gesamtmenge von zu prüfenden keramischen Komponenten, als sogenannte Probekomponenten mit einem Riss zur Ermittlung der Risszähigkeit nach dem beschriebenen Verfahren versehen werden. Diese Probekomponenten können dann später aussortiert werden und nicht für einen Einsatz verwendet werden. Bei der Gesamtmenge der zu prüfenden keramischen Komponenten kann es sich beispielsweise um eine Menge von mehr als 1000 keramischen Komponenten handeln. Ein Anteil von Probekomponenten, der zur Ermittlung der Risszähigkeit mit einem Riss versehen wird, kann aus der Gesamtmenge entnommen sein. Bei dem Anteil kann es sich beispielsweise um wenigsten 10, 15, 20, oder dreißig keramische Komponenten handeln. Der Anteil kann beispielsweise kleiner sein als 100, 90, 80 oder 70 keramische Komponenten.

Ergänzend oder alternativ kann das optische Überprüfen der Komponente auf Risse mittels einer schwarzen Farbe erfolgen. Es kann sich dabei beispielsweise um eine Risseindringfarbe und/oder eine Druckerfarbe handeln. Bei manchen Ausführungsbeispielen können damit Risse in Komponenten, die $Al_2O_3$ oder $ZrO_2$ umfassen, ausreichend gut erkannt werden. Eventuell kann die verwendete Farbe je nach Werkstoff der Komponente unterschiedlich sein, bei $Si_3N_4$ kann beispielsweise eine Fluoreszenzfarbe verwendet werden, die im UV-Licht fluoresziert.

Ergänzend oder alternativ kann das Verfahren auch bei Komponenten durchgeführt werden kann, die einen anderen keramischen Werkstoff als $Si_3N_4$ umfassen, beispielsweise SiAlON (Silizium-Aluminium-Oxy-Nitrid), SiC (Siliciumcarbid), $Al_2O_3$ (Aluminiumoxid), $ZrO_2$ (Zirconiumoxid), ZTA (Zirkoniumoxidverstärktes Aluminiumoxid), ATZ (aluminiumoxidverstärktes Zirkonoxid) oder dergleichen.

Offenbart wird auch auch eine keramische Komponente, die mit einem Verfahren nach einem der vorhergehenden Ansprüche auf eine Einsatztauglichkeit überprüft wurde. Bei manchen Ausführungsbeispielen kann dadurch ermöglich werden, dass die Komponente maximal Risse aufweist, die die kleiner sind als der Grenzwert. Bei manchen Ausführungsbeispielen kann so eine Komponente bereitgestellt werden, bei der ein Versagen in einem bestimmungsgemäßen Betrieb zumindest unwahrscheinlich ist.

Ergänzend kann die keramische Komponente als Werkstoff $Si_3N_4$, SiAlON, SiC, $Al_2O_3$, $ZrO_2$, oder deren Mischungen umfassen. Unter Umständen kann wenigstens einer dieser Werkstoffe wenigstens 50 Gewichts% eines Gesamtgewichts der Komponente ausmachen. Eventuell kann die Komponente auch vollständig aus einem dieser Werkstoffe hergestellt sein.

Ergänzend oder alternativ kann die keramische Komponente eine Risszähigkeit aufweisen., die größer oder gleich 4 MPaJm ist. Bei manchen Ausführungsbeispielen können dadurch besonders widerstandfähige Komponenten bereitgestellt werden.

Ergänzend oder alternativ kann die keramische Komponente an ihrer Oberfläche zumindest abschnittsweise eine Rauigkeit aufweist, die kleiner ist, als 15 $\mu$m 10 $\mu$m, 5 $\mu$m, 1 $\mu$m, 0.8 $\mu$m, 0.5 $\mu$m 0.2 $\mu$m, 0.1 $\mu$m, 0.05 $\mu$m, 0.05 $\mu$m, 0.01 $\mu$m, 0.008 $\mu$m, 0.005 $\mu$m oder 0.001 $\mu$m. Bei manchen Ausführungsbeispielen kann die Komponente dadurch besonders für ihren Einsatzzweck, beispielsweise als Lagerkomponente oder als Implantat ausgebildet sein. Ergänzend oder alternativ kann die Komponente die Rauigkeit an ihrer gesamten Oberfläche oder an einer Fläche, die wenigstens 50%, 60%, 70%, 80%, 90%, 95% oder 99% einer Gesamtoberfläche der Komponenten ausmacht, aufweisen.

Die in der vorstehenden Beschreibung, den nachfolgenden Ansprüchen und den beigefügten Figuren offenbarten Ausführungsbeispiele sowie deren einzelne Merkmale können sowohl einzeln wie auch in beliebiger Kombination für die Verwirklichung eines Ausführungsbeispiels in ihren verschiedenen Ausgestaltungen von Bedeutung sein und implementiert werden.

So zeigen die Figuren schematisch die nachfolgenden Ansichten.

Fig. 1      zeigt eine schematische Darstellung eines Verfahrens zur Prüfung einer keramischen Komponente gemäß einem Ausführungsbeispiel;

Fig. 2 a      zeigt eine schematische Darstellung einer Komponente, die mit dem erfindungsgemäßen Verfahren geprüft ist; und

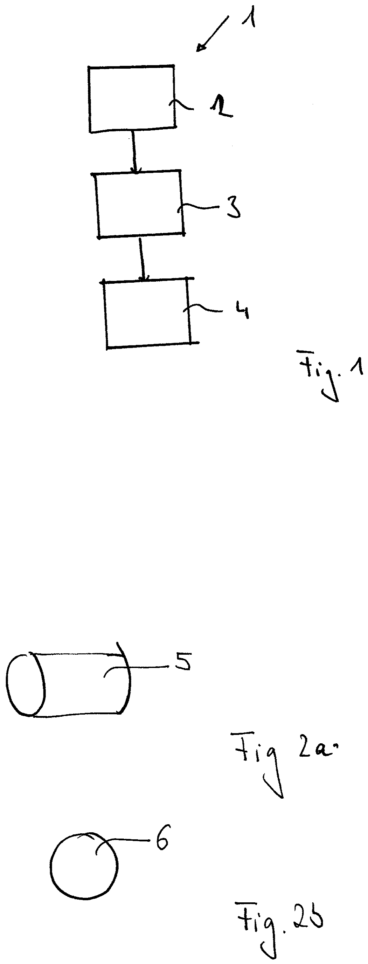

Fig. 2 b      zeigt eine schematische Darstellung einer weiteren Komponente, die mit dem erfindungsgemäßen Verfahren geprüft ist.

Bei der nachfolgenden Beschreibung der beigefügten Darstellungen bezeichnen gleiche Bezugszeichen gleiche oder vergleichbare Komponenten. Ferner werden zusammenfassende Bezugszeichen für Komponenten und Objekte verwendet, die mehrfach in einem Ausführungsbeispiel oder in einer Darstellung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt.

Fig. 1 zeigt ein Verfahren 1 zur Prüfung einer keramischen Komponente auf eine Einsatztauglichkeit. Dazu wird in einem ersten Vorgang 2 die Komponente auf eine erste Temperatur temperiert. Anschließend wird in einem zweiten Vorgang 3 die Komponente auf eine zweite Temperatur temperiert. Eine Temperaturdifferenz zwischen der ersten Temperatur und der zweiten Temperatur wird dabei auf Basis einer minimalen Risszähigkeit ermittelt. In einem dritten Vorgang 4 wird die Komponente auf Risse überprüft.

Bei manchen Ausführungsbeispielen kann die Komponente so lange auf die erste Temperatur erwärmt werden, bis der erwärmte Bereich oder die ganze Komponente homogen die erste Temperatur aufweist. Anschließend kann die Komponente rasch abgekühlt werden. Bei manchen Ausführungsbeispielen können dadurch einfache geometrische Rahmenbedingungen für die Berechnung der Temperaturdifferenz erreicht werden. Alternativ kann die Einstellung einer konstanten bzw. homogenen ersten Temperatur in der zu prüfenden Komponente entfallen. Es kann beispielsweise nur die Oberfläche oder eine Randschicht auf die erste Temperatur erwärmt und anschließend rasch abgekühlt werden.

Bei Ausführungsbeispielen, bei denen die Thermoschockbehandlung bzw. das Prüfverfahren aus einem raschen Aufheizen besteht, also die erste Temperatur kleiner ist als die zweite Temperatur, kann ebenfalls eine homogene Temperierung der Komponenten oder nur ein Temperieren der Randschichten ausgeführt werden. Ein rasches Aufheizen kann beispielsweise bei Komponenten gewählt werden, bei denen in einem Betrieb innenliegende Zugspannungen auftreten können, die in einem äußeren Bereich zu Druckspannungen führen können. Bei Komponenten, bei denen nur eine im Einsatz speziell beanspruchte Stelle zu prüfen ist, kann eventuell auch nur diese Stelle der Komponente mit der ersten Temperatur und dann der zweiten Temperatur temperiert werden. Beispielsweise kann ein bereichsweises rasches Aufheizen mit einem leistungsstarken Laser erfolgen.

Um die Risse in dem dritten Vorgang 3 zu detektieren können unterschiedliche Wege gewählt werden. Die Risse können grundsätzlich auf beliebige Art detektiert werden, beispielsweise akustisch, durch Schwingungsanalysen oder optisch. Risse, die nicht bis an die Oberfläche reichen, können beispielsweise mit Ultraschall oder Röntgenstrahlung detektiert werden.

Für eine optische Überprüfung können die Risse eventuell mit einer Risseindringfarbe kenntlich gemacht werden. Dabei wird die zu prüfende Komponente in eine gefärbte, beispielsweise schwarze Flüssigkeit, zum Beispiel Druckerfarbe getaucht. Die gefärbte Flüssigkeit verbleibt nach Entnahme des Körpers aus der Flüssigkeit in den Rissen. Eventuell kann es sich auch um eine fluoreszierenden Risseindringfarbe handeln. Die Risse können dann beispielsweise unter ultraviolettem Licht sichtbar gemacht werden. Egal mit welchem Verfahren die Risse detektiert werden, Komponenten die Risse aufweisen, die wenigstens einen Grenzwert überschreiten, können aussortiert werden.

Das Verfahren gemäß Ausführungsbeispielen kann sich beispielsweise dazu eigenen, in einer laufenden Produktion eingesetzt zu werden. Hierfür kann vorgesehen sein, dass die Komponente in einem Ofen im Durchlauf auf die erste Temperatur erwärmt wird. Dazu kann beispielsweise ein Fördermittel vorgesehen sein, mit dem die Komponente durch den Ofen transportiert wird. Anschließend kann die keramische Komponente mit dem Fördermittel zu einem Abschreckmittel geführt werden, um auf die zweite Temperatur temperiert zu werden. Als Abschreckmittel eignet sich zum Beispiel eine Flüssigkeit, beispielsweise Wasser oder Öl. Des Weiteren kann als Abschreckmittel auch ein Gas eingesetzt werden. Bei einer Abschreckung mit Gas kann das Gas mit erhöhtem Druck auf den Körper geführt werden, zum Beispiel mit einem Druck von 2 bar oder mehr. Bei anderen Ausführungsbeispielen kann die Komponente in dem ersten Vorgang 2 auch bereichsweise oder vollständig mit einem Gasbrenner erwärmt und anschließend in dem zweiten Vorgang 3 mit einem Luft jet abgekühlt werden.

Wird die keramische Komponente mit einem Fördermittel zu der Flüssigkeit als Abschreckmittel geführt, können die Komponenten in das Flüssigkeit fallen gelassen werden, um die Thermoschockbehandlung zu erreichen. Die Flüssigkeit weist dabei die zweite Temperatur auf. Alternativ kann die Komponente auch in das Fluid getaucht werden.

Bei manchen Ausführungsbeispielen kann das Temperieren auf die erste und die zweite Temperatur in einer einzelnen Vorrichtung durchgeführt werden, beispielsweise wenn mit einem Gas für eine Abschreckung von einer ersten Temperatur gearbeitet wird. Eventuell kann dazu eine kombinierte Erwärmungs- und Abschreckanlage eingesetzt werden. Ein Beispiel hierfür ist die Erwärmung in einem Vakuumhärteaggregat, wobei bei der Erwärmung mit oder ohne Vakuum gearbeitet werden kann, eventuell eine mit anschließender Hochdruckgasab schreckung.

Bei manchen Ausführungsbeispielen kann bei dem Temperieren ein inhomogenes Temperaturfeld an und/oder in der Komponente eingestellt werden. Bei manchen Ausführungsbeispielen kann sich dadurch, ein rasches Erwärmen der Komponente in Kombination mit einem raschen Abkühlen erreicht werden. Die Thermoschockbehandlung kann sich auf eine oberflächennahe Zone der Komponente oder die gesamten Komponente beziehen. Möglich ist es dabei auch, dass die Thermoschockbehandlung in einem Bereich durchgeführt wird, in welchem eine maximale Belastung der Komponente im Einsatz gegeben ist. Es können unter Umständen auch Kanten eines Körpers der Thermoschockbehandlung unterworfen werden.

Die Fig. 2 a und 2 b zeigen schematische Darstellungen von Komponenten 5 und 6 die mit dem Verfahren 1 geprüft wurden. Bei der Komponente 5 handelt es sich um eine Zylinderrolle und bei der Komponente 6 um eine Kugel. Die Komponenten 5 und 6 können zum Beispiel als Wälzkörper dienen. Die Komponenten 5 und 6 weisen als keramisches Material wenigstens einen der Werkstoffe $Si_3N_4$, SiAlON, SiC, $Al_2O_3$, $ZrO_2$, ZTA oder ATZ auf.

Mit dem Verfahren 1 wie können aber auch alle möglichen anderen beispielsweise keramische Komponenten geprüft werden. Es kann sich dabei beispielsweise um Komponenten handeln, die in Wälz-, Roll- oder Gleitanwendungen verbaut werden. Hierfür werden die Komponenten wie beschrieben, einer Prüfung mittels Thermoschock unterzogen und anschließend beispielsweise mit Risseindringfarbe beaufschlagt werden, um überkritisch lange Risse zu detektieren und damit schadhafte bzw. nicht einsatztaugliche Komponenten ausscheiden zu können.

**Bezugszeichenliste**

1    Verfahren
2    Temperieren
3    Temperieren
4    Temperieren
5    Komponente
6    Komponente

**Patentansprüche**

**1.** Verfahren (1) zur Prüfung einer keramischen Komponente (5) auf ihre Einsatztauglichkeit umfassend:

Ermitteln einer Temperaturdifferenz zwischen einer ersten Temperatur und einer zweiten Temperatur;
Temperieren (2) der Komponente (5) auf die erste Temperatur;
Temperieren (3) der Komponente (5) auf die zweite Temperatur;
Überprüfen (4) der Komponente auf Risse,
**gekennzeichnet durch**
ein Überprüfen der Einsatztauglichkeit der Komponente (5), wobei die Komponente (5) als einsatztauglich betrachtet wird, indem sie maximal Risse aufweist, die kleiner sind als ein Grenzwert, wobei der Grenzwert für eine Größe festgelegt worden ist, ab der ein Riss als kritisch betrachtet wird, wobei es sich bei der Größe des Grenzwerts um eine Länge, eine Breite und/oder eine Tiefe des Risses handelt,

wobei die Temperaturdifferenz auf Basis einer minimalen Risszähigkeit ermittelt wird, wobei es sich bei der minimalen Risszähigkeit um einen von einem Benutzer oder Anwender vorgegebenen Wert handelt, den die Komponente (5) aufweisen soll, um den Einsatzzweck zu erfüllen.

2. Verfahren nach Anspruch 1, wobei ein Wert für die minimale Risszähigkeit aus einer Tabelle entnommen wird.

3. Verfahren nach einem der vorhergehenden Ansprüche, wobei die Risszähigkeit basierend auf folgender Tabelle bestimmt wird.

| | $h_f$ = 75000 Wm$^{-2}$K$^{-1}$ | | | $h_f$ = 100000 Wm$^{-2}$K$^{-1}$ | | |
|---|---|---|---|---|---|---|
| | Bi | Ksurv | Kfrac | Bi | Ksurv | Kfrac |
| | - | MPa m$^{1/2}$ | MPa m$^{1/2}$ | - | MPa m$^{1/2}$ | MPa m$^{1/2}$ |
| Set 1, D=12.7 mm, HK10 | 22.0 | 5.3 ± 0.3 | 5.5 ± 0.3 | 29.3 | 5.7 ± 0.3 | 5.9 ± 0.3 |
| Set 2, D=5.55 mm, HK10 | 10.2 | 5.5 ± 0.3 | 5.9 ± 0.3 | 13.7 | 6.0 ± 0.3 | 6.4 ± 0.3 |
| Set 3, D=5.55 mm, HK7 | 10.6 | 5.7 ± 0.1 | 6.0 ± 0.2 | 14.2 | 6.2 ± 0.1 | 6.5 ± 0.2 |

wobei $h_f$ den Wärmeübergangskoeffizient, Bi eine dimensionslose Biotzahl, $K_{surv}$ die Risszähigkeit ohne Risswachstum, und $K_{frac}$ die Risszähigkeit mit Risswachstum bezeichnet, und HK den Knoop Indenter angibt.

4. Verfahren nach einem der vorhergehenden Ansprüche, wobei das Überprüfen der Risse mittels optischer Überprüfung erfolgt, wobei die Risse mit einer Risseindringfarbe, insbesondere mittels einer schwarzen Farbe, kenntlich gemacht werden.

5. Verfahren nach einem der vorhergehenden Ansprüche, ferner umfassend, Ermitteln einer Risszähigkeit an einer Probekomponente, die in Form, Größe und Material der keramischen Komponente entspricht.

**Claims**

1. Method (1) for testing a ceramic component (5) for its serviceability, comprising:

ascertaining a temperature difference between a first temperature and a second temperature;
conditioning (2) the component (5) to the first temperature;
conditioning (3) the component (5) to the second temperature;
inspecting (4) the component for cracks,
**characterized by**
an inspection of the serviceability of the component (5), wherein the component (5) is deemed to be serviceable by virtue of having at most cracks which are smaller than a limiting value, the limiting value having been specified for a size beyond which a crack is deemed to be critical, the size of the limiting value comprising a length, a width and/or a depth of the crack,
wherein the temperature difference is ascertained on the basis of a minimal fracture toughness, the minimal fracture toughness being a value which is mandated by a user or operator and which the component (5) is to have in order to fulfil the intended service.

2. Method according to Claim 1, wherein a value for the minimal fracture toughness is taken from a table.

3. Method according to either of the preceding claims, wherein the fracture toughness is determined on the basis of the following table:

| | $h_f$ = 75000 Wm$^{-2}$K$^{-1}$ | | | $h_f$ = 100000 Wm$^{-2}$K$^{-1}$ | | |
|---|---|---|---|---|---|---|
| | Bi | $K_{surv}$ | $K_{frac}$ | Bi | $K_{surv}$ | Kfrac |
| | - | MPa m$^{1/2}$ | MPa m$^{1/2}$ | - | MPa m$^{1/2}$ | MPa m$^{1/2}$ |
| Set 1, D,=12.7 mm, HK10 | 22.0 | 5.3 ± 0.3 | 5.5 ± 0.3 | 29.3 | 5.7 ± 0.3 | 5.9 ± 0.3 |
| Set 2, D=5.55 mm, HK10 | 10.2 | 5.5 ± 0.3 | 5.9 ± 0.3 | 13.7 | 6.0 ± 0.3 | 6.4 ± 0.3 |
| Set 3, D=5.55 mm, HK7 | 10.6 | 5.7 ± 0.1 | 6.0 ± 0.2 | 14.2 | 6.2 ± 0.1 | 6.5 ± 0.2 |

wherein $h_f$ denotes the heat transfer coefficient, Bi denotes a dimensionless biot number, $K_{serv}$ denotes the fracture toughness without crack propagation and $K_{frac}$ denotes the fracture toughness with crack propagation, and HK indicates the Knoop Indenter.

**4.** Method according to any of the preceding claims, wherein the cracks are inspected by means of optical inspection, the cracks being visualized with a crack-penetrating ink, more particularly by means of a black ink.

**5.** Method according to any of the preceding claims, further comprising ascertaining a fracture toughness on a specimen component which in shape, size and material corresponds to the ceramic component.

**Revendications**

**1.** Procédé (1) de contrôle d'aptitude à l'emploi d'un composant céramique (5), comprenant :

la détermination d'une différence de température entre une première température et une deuxième température ;
la thermorégulation (2) du composant (5) à la première température ;
la thermorégulation (3) le composant (5) à la deuxième température ;
la vérification (4) du composant s'agissant de fissures, **caractérisé par**
la vérification d'aptitude à l'emploi de la pièce (5), la pièce (5) étant considérée comme apte à l'emploi en ce qu'elle présente un maximum de fissures inférieures à une valeur limite, la valeur limite ayant été définie pour une taille à partir de laquelle une la fissure est considérée comme critique, la taille de la valeur limite étant une longueur, une largeur et/ou une profondeur de la fissure,
la différence de température étant déterminée sur la base d'une ténacité minimale de la fissure, la ténacité minimale de la fissure étant une valeur spécifiée par un opérateur ou un utilisateur, que le composant (5) doit présenter afin de répondre à l'utilisation prévue.

**2.** Procédé selon la revendication 1, dans lequel une valeur pour la ténacité minimale à la rupture est tirée d'un tableau.

**3.** Procédé selon l'une quelconque des revendications précédentes, dans lequel la ténacité à la rupture est déterminée sur la base du tableau suivant.

| | $h_f$ = 75000 Wm$^{-2}$K$^{-1}$ | | | hf = 100000 Wm$^{-2}$K$^{-1}$ | | |
|---|---|---|---|---|---|---|
| | Bi | Ksurv | $K_{frac}$ | Bi | $K_{surv}$ | $K_{frac}$ |
| | - | MPa m$^{1/2}$ | MPa m$^{1/2}$ | - | MPa m$^{1/2}$ | MPa m$^{1/2}$ |
| Ensemble 1, D=12,7 mm, HK10 | 22,0 | 5,3 ± 0,3 | 5,5 ± 0,3 | 29,3 | 5,7 ± 0,3 | 5,9 ± 0,3 |
| Ensemble 2, D=5,55 mm, HK10 | 10,2 | 5,5 ± 0,3 | 5,9 ± 0,3 | 13,7 | 6,0 ± 0,3 | 6,4 ± 0,3 |
| Ensemble 3, D=5,55 mm, HK7 | 10,6 | 5,7 ± 0,1 | 6,0 ± 0,2 | 14,2 | 6,2 ± 0,1 | 6,5 ± 0,2 |

dans lequel $h_f$ est le coefficient de transfert de chaleur, Bi est un nombre de Biot sans dimension, $K_{surv}$ est la ténacité à la rupture sans croissance de fissure, et $K_{frac}$ est la ténacité à la rupture avec croissance de fissure, et HK est le pénétrateur Knoop.

**4.** Procédé selon l'une quelconque des revendications précédentes, dans lequel le contrôle des fissures s'effectue par inspection visuelle, les fissures étant repérées par une couleur de pénétration des fissures, notamment au moyen d'une couleur noire.

5. Procédé selon l'une quelconque des revendications précédentes, comprenant en outre la détermination d'une ténacité à la rupture sur un composant échantillon qui correspond en forme, taille et matériau au composant céramique.

Fig. 1

Fig 2a.

Fig. 2b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010017351 A1
- JP H0886731 A